# EUROPEAN PATENT SPECIFICATION

(11) **EP 1 881 838 B1**
(45) Date of publication and mention of the grant of the patent: **18.07.2012**
(21) Application number: 06741319.5
(22) Date of filing: 16.05.2006
(51) Int. Cl.: A61K 35/74, C12N 13/00, A61K 39/04, C12N 1/06

(54) **ANTITUMOR AGENT ON THE BASE OF BCG VACCINE, METHOD FOR ITS PREPARATION AND ITS USE**
ANTITUMORMITTEL AUF DER BASIS DES IMPFSTOFFS BGC, HERSTELLUNGSVERFAHREN DAFÜR UND VERWENDUNG DAVON
AGENT ANTITUMORAL BASE SUR UN VACCIN BCG, METHODE DE PREPARATION ET UTILISATION

(30) Priority: 18.05.2005 BG 10916005
(43) Date of publication of application: 30.01.2008
(73) Proprietor: Hadjiev, Spartak, 4000 Plovdiv (BG); Hadjieva, Nasia, 1750 Sofia (BG); Petrov, Mihail, 1750 Sofia (BG)
(72) Inventor: HADJIEVA, Dora, 4000 Plovdiv (BG); RACHKOVSKA, Zoia, 4000 Plovdiv (BG); HADJIEV, Spartak, 4000 Plovdiv (BG); HADJIEVA, Nasia, 1750 Sofia (BG); PETROV, Mihail, 1750 Sofia (BG)
(74) Representative: Kosseva, Radislava Andreeva
(86) International application number: PCT/BG2006/000011
(87) International publication number: WO 2006/122380

(56) References cited:
- EP-A- 0 710 484
- WO-A-95/26742
- ZBAR B ET AL: "PREVENTION OF TUMOR GROWTH AFTER INTRA DERMAL INJECTION OF BCG EXTRACTS A COMPARISON OF RESULTS IN STRAIN 2 GUINEA-PIGS FROM THE NATIONAL INSTITUTES OF HEALTH AND FROM THE NATIONAL JEWISH HOSPITAL AND RESEARCH CENTER" JOURNAL OF THE NATIONAL CANCER INSTITUTE, vol. 56, no. 2, 1976, pages 443-444, XP009071871
- HADJIEV S ET AL: "The effect of BCG treatment on gastric carcinoma. Three case reports" FOLIA MEDICA 1981 BULGARIA, vol. 23, no. 1, 1981, pages 59-65, XP009071850
- HADJIEV S ET AL: "The effect of dosage and treatment scheme on results from treatment of lung cancer with BCG and F70." FOLIA MEDICA. 1983, vol. 25, no. 4, 1983, pages 8-15, XP009071849 ISSN: 0204-8043

## Description

### Technical area

The invention relates to a method for obtaining of antitumor composition on the basis of BCG vaccine, the antitumor composition and its application for manufacture a medicine for treatment of patients diagnosed with oncological diseases.

### Background of the invention

The results of a number of scientific studies published over the past several years show that the antitumor properties of the Mycobacteriaceae family are due to active agents it contains or produces.

Thus, US patent 6, 274, 356 describes a carbohydrate complex, in essence representing a complex of polysaccharides, extracted from Mycobacterium vaccae, which shows high antitumor activity in the treatment of different oncological diseases. The method of preparation of the carbohydrate complex has also been described as a series of actions, involving obtaining precipitates and dialisates by extraction and purification.

### Summary of the invention

The problem of the present invention is obtaining of a composition with antitumor activity based on the BCG vaccine, which gives maximum opportunity for the action of the antitumor factor of the BCG vaccine in order to terminate the rapid and uncontrolled proliferation of tumor cells in an environment of reduced toxicity and blocking of the inhibitive effect of tumor cells on normal cells in the body, as well the method of obtaining of BCG-based composition with antitumor activity and its use for manifacture of a medicine for treatment of solid tumours..

According to the invention this problem was solved with a method for preparation of a composition with antitumor activity as per the invention. The essence of the method lies in obtaining two different fractions, respectively termed Fraction 1 - "Live BCG Bacteria" and Fraction 2 - "Extract-filtrate from BCG vaccine ", which under specific processing conditions are mixed in a certain proportion.

Fraction 1 is made from a standard ampoule of 500 µg dry substance of BCG vaccine, the shelf-life (exp.date) of which must not have expired. Following the instructions the ampoule is diluted with standard solvent whilst strictly observing sterility rules in a sterile laboratory box, treated by UV lamps for 18-24 hours in advance. The resulting suspension is additionally diluted with physiological solution or distilled water up to a concentration of 100 to 10 µg/ml BCG. This suspension is diluted again until a concentration of 10 to 0.001 µg/ml is obtained (accepted as 10 provisional units according in line with the invention).

The content of live BCG bacteria is controlled by periodic microbiological samples on Leuvenstein-Jensen media for tuberculosis bacteria containing (in g/l to double distilled water):

| | |
|---|---|
| L-Aspargine | 3.6 |
| Monocalcium phosphate | 2.4 |
| Magnesium sulfate | 0.24 |
| Magnesium citrate | 0.6 |
| Potato flour | 30.0 |
| Malachite green | 0.4 |
| Glycerol | 12.0 ml |
| Eggs | 1 litre |
| pH 7.2 ± 0.2 at 25 °C | |

Growth is controlled by the presence of specific colonies and specific morphology of the bacteria stained by Ziehl-Nielsen and examined under the microscope.

Fraction 2 is generated from standard BCG vaccine by adding 3 to 5ml physiological solution or 4 to 6 ml distilled water and ultrasound treatment for 8 to 12 minutes. The resulting suspension is left for a period of 2 to 60 days, preferably 2 to 20 days in a refrigerator at a temperature of 4-8°C.

The content of live BCG bacteria is controlled by periodic microbiological samples on Leuvenstein-Jensen media for tuberculosis bacteria and the growth is demonstrated by the presence of specific colonies and specific morphology of the bacteria stained by Ziehl-Nielsen and examined under the microscope.

Between the 2^{nd} and the 60^{th} day the suspension is filtered though a sterile filter and the filtrate obtained is stored in a refrigerator at a temperature of 4 to 8°C as initial extract-filtrate, which contains no live BCG bacteria.

Fraction 2 is a clear solution without any visible impurities, colour or odour. The chemical components of Fraction 2 include proteins, nucleoproteides, free aminoacids, polysaccharides, and small quantity of fats with the ratio between individual ingredients varying uncontrollably.

After the control examination aimed to establish the presence of live bacteria, the two fractions are mixed at Fraction 1 to Fraction 2 ratio from 0.01:1 to 1:0.01.

Another object of the invention is based on BCG vaccine (BCG with Mycobacterium bovis as active substance, controlled by the manufacturer in compliance with WHO requirements regarding the physical, chemical and biological properties of the bacterial strains and of the final product), antitumor composition obtaining by the above described method as per invention, which has a powerful inhibitive effect on tumor cells and has demonstrated no undesirable side effects.

The antitumor composition according to the invention is a mixture of two independent fractions the ratio between which may vary from 1:0.01 to 0.01:1.

Fraction 1 named "Live BCG Bacteria" contains a standard BCG vaccine, diluted once by physiological solution or distilled water to a concentration from 100 to 10 µg/ml and secondly diluted by physiological solution from 10 to 0.001 µg/ml.

Our definition of standard BCG vaccine solution means 1 ampoule BCG vaccine containing 500 µg dry substance, diluted by 1 ml distilled water used as standard solvent.

Fraction 2 termed "Extract-filtrate from BCG vaccine" contains standard solution of BCG vaccine, diluted by 4-6 ml distilled water or 3-5 ml physiological solution.

Yet another aspect of the invention is the use of the BCG-vaccine based composition with antitumor activity. The antitumor composition as per the invention is used for the manufacture of a medicine for treatment of all forms of malignancies in all stages of the disease.

The medicine manufactured from antitumor acting composition /substance as per the invention is further applied for the treatment of various forms of cancer and specifically cancer of the throat, breast cancer, stomach cancer, liver cancer, cancer of the uterus, cancer of the colon, brain tumors, malignant melanoma, skin cancer and any other type of solid tumors, as well as of the metastases originating from various solid tumors in all systems of the human body.

Immediately after mixing the two fractions in the ratio determined as per the invention the medicine manufactured from BCG vaccine based antitumor composition is injected intradermally to patients with cancer in a ratio from 0.1 to 0.2 ml.

For the purpose of sustained use the antitumor composition as per the invention is transferred to an ice bath and inserted into vacuum ampoules of 0.2 ml.

### Advantages of the Invention

As per the invention the main advantages of the antitumor composition are:
(1) powerful effect on tumor cells and termination of their rapid and uncontrolled proliferation;
(2) powerful effect on metastases, stopping their growth and leading to their regression and disappearance;
(3) blocking the repressing effect of tumor cells on normal cells in the system;
(4) low toxicity;
(5) possibly acts as cell growth factors, controlling the non-differentiated proliferation of cancer cells;
(6) simplified method of obtaining the composition.

### Example for implementation of the invention

The invention can be illustrated by the examples below, which clarify it, without prejudice to the scope of its protection.

### Example 1

### Obtaining Fraction 1 - "Live BCG Bacteria"

One standard BCG vaccine ampoule (manufactured by BUL BIO National Center of Infectious and Parasitic Diseases, Sofia) within the designated shelf-life, containing 500 µg dry substance is opened in sterile laboratory box treated with UV lamps for 18 hours in advance. One ml of distilled water is added and the resulting suspension is diluted with physiological solution to a concentration of 100 µg BCG in 1 ml physiological solution. The resulting suspension is further diluted to 10 µg/ml BCG. The content of live BCG bacteria is controlled by periodic microbiological samples on Lowenstein-Jensen media for tuberculosis bacteria. The growth is demonstrated by the presence of specific colonies and specific morphology of the bacteria stained by Ziehl-Nielsen and examined under the microscope.

### Obtaining Fraction 2 - "Extract-filtrate from BCG vaccine "

One standard BCG vaccine vial is opened in a sterile laboratory box treated with UV lamps in advance. One ml of a standard vaccine solvent is added. Three ml physiological solution are added to the resulting suspension, which is then treated by ultrasound for 8 min and refrigerated at a temperature of 4 to 8 °C for 2 days. On the 1^{st} and the 2^{nd} day the suspension is periodically controlled by microbiological samples on Lowenstein-Jensen media and then filtered though sterile filter Falcon® where the pores are < 0.1 mm and the filtrate obtained is a ready-for-use Fraction 2.

### Final preparation of the antitumor composition

The two fractions obtained in the manner described above are mixed under sterile conditions in a sterile container in the ratio = Fraction 1 : Fraction 2 as 1 : 0.01 where the resulting mixture is a ready-for-use composition as per the invention.

The composition with antitumor activity as per the invention is applied to the patients strictly intradermally on the volar part of the hand as Mantoux in a quantity of 0.1 ml.

### Example 2

### Obtaining Fraction 1 - "Live BCG Bacteria"

One standard BCG vaccine ampoule within the designated shelf-life, containing 500 µg dry substance is opened in sterile laboratory box treated with UV lamps for 20 hours in advance and 1ml of distilled water is added. The resulting suspension is diluted with distilled water to a concentration of 50 µg/ml. The resulting suspension is additionally diluted with physiological solution to a concentration of 0.1 µg/ml.

The presence of live BCG bacteria is controlled through periodic microbiological samples as described in Example 1.

### Fraction 2 - "Extract-filtrate from BCG vaccine"

One standard BCG vaccine vial is opened in a sterile laboratory box treated with UV lamps in advance. Then 1 ml of standard vaccine diluent is added. To the resulting suspension 4 ml physiological solution is added. The resulting suspension is then treated by ultrasound for 9 min and refrigerated at a temperature of 4 to 8°C for 10 days whilst periodically controlled for the presence of live bacteria as described in Example 1. On the 10^{th} day the suspension is filtered though a sterile filter Falcon® where the pores are < 0.1 mm and the filtrate obtained is a ready-for-use Fraction2.

### Final preparation of the antitumor composition

The two fractions obtained in the manner described above are mixed under sterile conditions in a sterile container in the ratio = Fraction 1 : Fraction 2 equal to 1 : 0.1 where the resulting mixture is a ready-for-use composition as per the invention.

The composition with antitumor activity as per the invention is applied strictly intradermally on the volar part of the hand as Mantoux in a quantity of 0.1 ml.

### Example 3

### Obtaining Fraction 1 - "Live BCG Bacteria"

One standard BCG vaccine ampoule is diluted with standard vaccine solvent in a sterile laboratory box, treated by UV lamp for 22 hours in advance, as described in Example 1. The obtained suspension is diluted with physiological solution to a concentration of 10 µg/ml. The resulting suspension is additionally diluted with physiological solution to a concentration of 0.001 µg/ml.

The presence of live BCG bacteria is controlled as described in Example 1 above.

### Fraction 2 - "Extract-filtrate from BCG vaccine"

One standard BCG vaccine vial is opened in a sterile laboratory box treated with UV lamps in advance and after that 1 ml distilled water is added. The obtained suspension is then diluted by 5 ml physiological solution, which is treated by ultrasound for 10 min and refrigerated at a temperature of 4 to 8°C for 20 days whilst periodically controlled for the presence of live bacteria as described in Example 1. On the 20^{th} day the suspension is filtered though sterile filter Falcon® where the pores are <0.1 mm. The resulting filtrate doesn't contain live BCG bacteria and is a ready-for-use Fraction 2.

### Final preparation of the antitumor composition

The two fractions obtained in the manner described above are mixed under sterile conditions in a sterile container in the ratio = Fraction 1 : Fraction 2 equal to 1 : 1. During transportation the suspension is transferred to an ice bath and then into vacuum ampoules of 0.2 ml and refrigerated at 4-8°C.

The resulting composition with antitumor activity as per the invention is fit for use within 1 month of production.

The composition with antitumor activity as per the invention is applied to the patients with oncological disease strictly intradermally on the volar part of the hand as Mantoux in a quantity of 0.1ml.

### Example 4

### Obtaining Fraction 1 - "Live BCG Bacteria"

In a sterile laboratory box treated by UV lamp for 24 hours in advance one standard ampoule of BCG vaccine is diluted with a standard vaccine solvent as described in Example 1. The obtained suspension is diluted with physiological solution to a concentration of 10 µg/ml. The resulting suspension is additionally diluted with physiological solution to a concentration of 0.001 µg/ml.

The presence of live BCG bacteria is controlled as described in Example 1 above.

### Obtaining Fraction 2 - "Extract-filtrate from BCG vaccine "

One standard BCG vaccine vial is opened in a sterile laboratory box treated with UV lamps in advance and after that 1 ml distilled water is added. Then the obtained suspension is diluted with 6 ml distilled water which is treated by ultrasound for 12 min and refrigerated at a temperature of 4 to 8°C for 40 days whilst periodically controlled for the presence of live bacteria as described in Example 1. On the 40^{th} day the suspension is filtered though a sterile filter Falcon® where the pores are < 0.1 mm. The resulting filtrate doesn't contain live BCG bacteria and is a ready-for-use Fraction 2.

### Final preparation of the antitumor composition

The two fractions obtained in the manner described above are mixed under sterile conditions in a sterile container in the ratio = Fraction 1 : Fraction 2 equal to 0.1 : 1.

The composition with antitumor activity as per the invention is applied strictly intradermally on the volar part of the hand as Mantoux in a quantity of 0.1 ml.

### Example 5

### Obtaining Fraction 1 - "Live BCG Bacteria"

In a sterile laboratory box treated by UV lamp for 23 hours in advance one standard ampoule of BCG vaccine is diluted with standard vaccine solvent as described in Example 1. The obtained suspension is diluted with distilled water to a concentration of 20 µg/ml. The resulting suspension is additionally diluted with physiological solution to a concentration of 0.01 µg/ml.

The presence of live BCG bacteria is controlled as described in Example 1 above.

### Obtaining Fraction 2 - "Extract-filtrate from BCG vaccine"

One standard BCG vaccine vial is opened in a sterile laboratory box treated with UV lamps in advance and 1 ml distilled water is added. Then, the obtained suspension is diluted with 4 ml physiological solution, which is treated by ultrasound for 10 min and refrigerated at a temperature of 4 to 8°C for 60 days whilst periodically controlled for the presence of live bacteria as described in Example 1. On the 60^{th} day the suspension is filtered though a sterile filter Falcon® where the pores are < 0.1 mm. The resulting filtrate doesn't contain live BCG bacteria and is a ready-for-use Fraction 2.

### Final preparation of the antitumor composition

The two fractions obtained in the manner described above are mixed in a sterile container under sterile conditions in the ratio = Fraction 1 : Fraction 2 equal to 0.01 : 1.

The composition with antitumor activity as per the invention is applied strictly intradermally on the volar part of the hand as Mantoux in a quantity of 0.2 ml.

### Examples of clinical tests of the composition with antitumor activity as per the invention

### In Brain Tumors

### Example 6

Patient B. V., 57 year-old male diagnosed with Craniopharingeoma. Partial resection performed. At that time the patient complained of severe headache, disturbances of the conscious, visual impairments and motor weakness when moving. The ensuing treatment was terminated due to deterioration of symptoms. Soon after the first administration of the composition with antitumor activity as per the invention visible improvement in the patient's condition could be observed to the gradual complete disappearance of symptoms. The patient is now leading a normal, healthy life.

### Example 7

Patient, M. G., 24 year-old male diagnosed with Meduloblastoma causing spastic paralysis of the right leg and full patient immobilization. Within 6 months after the first administration of the antitumor composition as per the invention the paralysis gradually disappears and full recovery is gradually achieved.

### Example 8

Patient S.T. 21 year-old female diagnosed with Ewing's Sarcoma. The therapy with the composition as per the invention commenced after surgical treatment and chemotherapy. At this stage crawling paralysis of the patient's leg is present and the patient was immobilized in a wheelchair. During the course of treatment with the antitumor composition as per the invention she regained the ability to walk on her own without assistance.

### In Cancer of the Urinary Bladder

### Example 9

Patient D.K., 51 year-old diagnosed with bladder cancer is subjected to Trans-Urethral Resection (TUR) every 3-4 months and subsequently went through surgery due to massive chematuria. Several months after administration of the antitumor composition as per the invention, the patient is in good condition and lives a normal life.

### Example 10

Patient A.A., 46 year-old male, diagnosed with bladder cancer in bad general condition due to profuse chematuria. Soon after administration of the antitumor composition as per the invention the patient's condition improved and no chematuria is presently observed. Several subsequent cystoscopies showed negative results for the presence of tumor cells.

### Example 11

Patient C.C., 57 year-old male, physician, diagnosed with bladder cancer. TUR is applied once in 2-3 months. After administration of the antitumor composition as per the invention the chematuria disappeared and the following cystoscopies showed negative results for the presence of tumor cells.

### In Skin Cancer

### Example 12

Patient N.T., diagnosed with melanoma malignum. After application of the antitumor composition the melanoma spot disappeared.

### Example 13

Patient G.G., diagnosed with Angiosarcoma with large tumor mass in the area of the armpit, which gradually disappeared after administration of the antitumor composition as per the invention leaving only a crust.

### Example 14

Patient D.D. diagnosed with Sarcoma and large tumor mass in the area of the arm. Following treatment with the antitumor composition as per the invention for several months the tumor disappeared leaving only a scar.

### In Lung Cancer

### Example 15

Patient I.T., male, diagnosed with Ca pulmonum

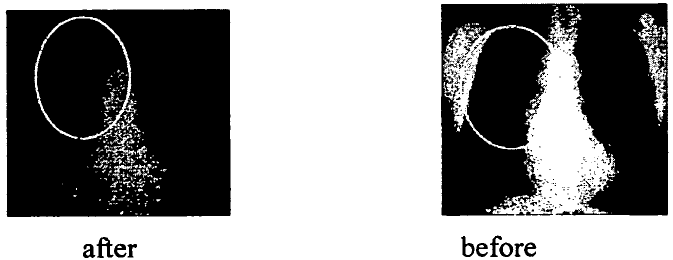

### Example 16

Patient M.G., male, diagnosed with Ca pulmonum

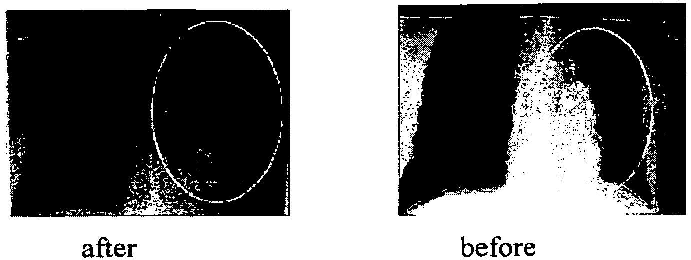

### Example 17

Patient N.R.D, female, diagnosed with Ca pulmonum

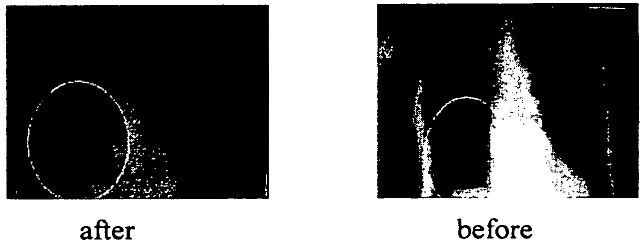

## Claims

1. A method for obtaining an antitumor composition comprising the mixing of two separate fractions prepared on the basis of a standard *Mycobacterium bovis* BCG vaccine, wherein
fraction 1, containing live bacteria, is obtained from a vial of standard BCG vaccine containing 500 µg dry substance suspended in 1 ml distilled water, by first diluting said suspension with a physiological solution or distilled water to a concentration of 100 to 10 µg/ml BCG, and additionally diluting said suspension with a physiological solution to a concentration of 10 to 0.001 µg/ml BCG
and
fraction 2, containing an extract-filtrate of BCG, obtained by:
- adding to the standard solution of 500 µg BCG vaccine in 1 ml distilled water and additional 3-5 ml of a physiological solution or 4-6 ml distilled water,
- treating the ensuing suspension with ultrasound for 8-12 minutes,
- refrigerating at 4-8°C for 2-60 days, preferably for 2-20, days, and
- passing the suspension through a sterile filter,
mixing fraction 1 and fraction 2 at a ratio between 0.01 : 1 to 1 : 0.01.

2. An antitumor composition on the basis of BCG vaccine, obtained through the method according to claim 1.

3. Use of antitumor composition on the basis of BCG vaccine, according to claim 2, for the manufacture of medicine meant for the treatment of solid tumors, such as cancer of the throat, breast cancer, stomach cancer, liver cancer, cancer of the uterus, cancer of the colon, skin cancer, brain tumors, malignant melanoma, as well as for the treatment of metastases in all systems of the human body, by means of intradermal application of the medicine in quantities between 0.1 and 0.2 ml.

## Patentansprüche

1. Ein Verfahren zur Erlangung einer Antitumor-Zusammensetzung umfasst das Mischen von zwei verschiedenen Fraktionen auf der Basis eines Standard-Mycobacterium bovis BCG-Impfstoff. Die erste Fraktion, mit lebenden Bakterien, wird aus einer Phiole mit Standard-BCG-Impfstoff mit 500µg Trockensubstanz - in 1 ml destilliertes Wasser suspendiert, indem zuerst Verdünnung des genannten Suspension mit einer physiologischen Lösung oder destilliertem Wasser zu einer Konzentration von 100 bis 10 µg/ml BCG, und
zusätzlich Verdünnung der Suspension mit einer physiologischen Lösung auf eine Konzentration von 10 bis 0,001 µg/ml BCG gemacht wird. Die zweite Fraktion enthält einen Extrakt-Filtrat von BCG, erhalten durch:
a. Hinzufügen zum Standard-Lösung von 500 µg BCG-Impfstoff in 1 ml destilliertem Wasser und zusätzliche 3-5 ml einer physiologischen Lösung oder 4-6 ml destilliertem Wasser,
b. Behandlung der daraus resultierenden Suspension mit Ultraschall für 8-12 Minuten,
c. Kühlung bei 4-8 ° C für 2-60 Tage, vorzugsweise für 2-20 Tage, und
d. Vergehen der Suspension durch einen sterilen Filter, Mischen die erste Fraktion und zweite Fraktion im Verhältnis von 0,01: 1 bis 1:0,01.

2. Ein Anti-Tumor-Zusammensetzung auf der Basis von BCG-Impfstoff, durch die Methode nach Anspruch 1 erhalten

3. Einsatz von Anti-Tumor-Zusammensetzung auf der Basis von BCG-Impfstoff, nach Anspruch 2, für die Herstellung von Arzneimittel zur Behandlung von soliden Tumoren, wie z. B. Kehlkopfkrebs, Brustkrebs, Magenkrebs, Leberkrebs, Krebs der Gebärmutter, Darmkrebs, Hautkrebs, Hirntumoren, malignes Melanom, als auch für die Behandlung der Metastasen in allen Systemen des menschlichen Körpers durch intradermale Anwendung des Medikaments in Dosen zwischen 0,1 und 0,2 ml.

## Revendications

1. Un procédé pour l'obtention d'une substance antitumorale comprenant le mélange de deux fractions séparées, préparée sur la base d'un vaccin standard de BCG Mycobacterium bovis, où la Fraction 1, qui contient des bactéries vivantes, est obtenue à partir d'un flacon de vaccin BCG standard contenant 500 µg de substance sèche en suspension dans 1 ml de l'eau distillée, diluée d'abord avec une solution physiologique ou d'eau distillée pour l'obtention d'une concentration de 100 à 10 µg/ml BCG, et diluée ensuite avec une solution physiologique jusqu'à l'obtention d' une concentration de 10 à 0,001 µg/ml de BCG ; et où la Fraction 2, qui contient un extrait-filtrat du BCG. est obtenue par:
- l'ajout à une solution standard de 500 µg de vaccin BCG diluée dans 1 ml d'eau distillée 3 à 5 ml supplémentaires de solution physiologique ou 4 à 6 ml d'eau distillée
- le traitement de la substance obtenue aux ultrasons pendant 8 à 12 minutes
- la réfrigération à 4 - 8°C pour 20 à 60 jours, de préférence pour les 2 à 20 jours et le filtrage de la substance à travers un filtre stérile
- le mélange des deux fractions, Fraction 1 et Fraction 2, dans un ratio, dans un rapport entre 0,01: 1 jusqu'à 1: 0,01.

2. Une substance antitumorale sur la base du vaccin BCG, obtenue par le procédé décrit dans l'affirmation 1.

3. Utilisation de la substance antitumorale basée sur le vaccin BCG, selon l'affirmation 2, pour la fabrication d'un médicament destiné au traitement de tumeurs solides, telles que le cancer de la gorge, le cancer du sein, le cancer de l'estomac, le cancer du foie, le cancer de l'utérus, le cancer du côlon, le cancer de la peau, des tumeurs au cerveau, le mélanome malin, ainsi que pour le traitement des métastases dans tous les systèmes du corps humain, par le biais de l'application intradermique du médicament dans des quantités comprises entre 0,1 et 0,2 ml.
